Europäisches Patentamt

European Patent Office

Office européen des brevets

(11) Publication number: **0 371 600**
**A1**

## (12) EUROPEAN PATENT APPLICATION

(21) Application number: **89310881.1**

(22) Date of filing: **23.10.89**

(51) Int. Cl.5: **G01N 33/84, C12Q 1/32**

(30) Priority: **29.11.88 JP 299551/88**

(43) Date of publication of application:
**06.06.90 Bulletin 90/23**

(84) Designated Contracting States:
**DE FR GB**

(71) Applicant: **ORIENTAL YEAST CO., LTD.**
**6-10, Azusawa 3-chome Itabashi-ku**
**Tokyo(JP)**

(72) Inventor: **Sunahara, Yoshiko**
**7, Toriishi 5-chome**
**Takaishi-shi Osaka-fu(JP)**
Inventor: **Takata, Shigeru**
**4-27, Minamiasahigaoka-cho**
**Tondabayashi-shi Osaka-fu(JP)**
Inventor: **Takagahara, Isamu**
**3-59, Fushimidai 5-chome Inagawa-cho**
**Kawabe-gun Hyogo-ken(JP)**

(74) Representative: **Arthur, John William et al**
**FITZPATRICKS 4 West Regent Street**
**Glasgow G2 1RS Scotland(GB)**

(54) Method of assaying magnesium.

(57) An assay of magnesium employing isocitrate dehydrogenase which increases its activity in a fixed relation with an increase of magnesium ions.

*FIG. 1*

ABSORBANCE ( Δ A340/min )

CONCENTRATION OF MAGNESIUM
( mg/dℓ )

EP 0 371 600 A1

## METHOD OF ASSAYING MAGNESIUM

### Field of the Invention:

This invention relates to a method of determining a small quantity of magnesium. More particularly, it is a method of assaying magnesium in the body fluids of man.

Magnesium occurs widely in the cells of animals and plants and is essential to their growth and life.

The quantity of magnesium in living bodies varies in an antagonistic relation to calcium, and its variation is observed in patients suffering from insufficiency of the central nervous system, heart, or kidney, acute pancreatitis, etc. Therefore, the assay of magnesium is becoming one of the important items of clinical examination

The method of this invention enables a quick and accurate assay of magnesium in body fluids, and thereby contributes greatly to clinical examination.

### Prior Art and Problems:

A colorimetric method which employs xylidyl blue to form a chelate is known for assaying magnesium. Atomic absorption spectrometry is another known method. Both of these methods, however, have drawbacks. The substance employed by the former method does not have a sufficiently high specificity for magnesium. The latter requires a special apparatus.

### Brief Description of the Drawings:

FIG. 1 shows the calibration curve of magnesium assay as obtained in Example 1, and

FIG. 2 shows the calibration curve of magnesium assay as obtained in Example 2.

These Examples will hereinafter be described.

### Means to Solve the Problems:

We, the inventors of this invention, have studied in detail the relations which may exist between magnesium and the activity of various kinds of enzymes, in order to find out a method which can determine the quantity of magnesium in body fluids quickly and accurately. As a result, we have completed this invention on the basis of our findings that isocitrate dehydrogenase requires magnesium or manganese ions to exhibit its activity and shows a higher activity with an increase of magnesium or manganese ions.

According to this invention, therefore, there is provided a method which relies upon the reaction of isocitrate dehydrogenase to determine the quantity of magnesium.

The method of this invention for assaying magnesium relies upon the nature of isocitrate dehydrogenase that is activity increases in a quantitative relation to an increase of magnesium ions.

More specifically, a sample of the body fluid to be examined is added to a reactant solution obtained by dissolving $NAD(P)^+$, isocitric acid and isocitrate dehydrogenase in an appropriate buffer solution, so that NAD(P)H may be formed as a product of their reaction and bring about an increase of absorbance at 340 nm. This increase of absorbance is determined by a rate assay, or alternatively, after a reaction stopper has been added to stop their reaction. An agent for chelating magnesium ions, or adenosine triphosphate (ATP), or both can be used as the reaction stopper.

Although isocitrate dehydrogenase is activated by manganese ions, too, it is possible to avoid any influence of manganese ions by adding to the reactant solution a chelating agent which does not trap magnesium ions, but selectively traps manganese ions, such as GEDTA.

This invention makes it possible to perform a highly specific assay of magnesium quickly and accurately by utilizing the activity of isocitrate dehydrogenase which increases with an increase of magnesium ions. The method of this invention can assay magnesium accurately even in a very small amount of sample.

The invention will now be described in further detail with reference to a couple of examples.

### Example 1

A standard solution containing 250 mg/dl of $Mg^{2+}$ was prepared by dissolving 42.3 mg of $MgCl_2 \cdot 6H_2O$ in 10 ml of purified water. The standard solution was diluted in various ways to make solutions containing 0, 5, 10, 15, 20, 25, 30, 35, 40, 45 and 50 mg/dl, respectively, of $Mg^{2+}$.

Then, 50 μl of each solution was added to 3 ml of a reactant solution of the composition shown below, and a variation of absorbance at 340 nm was determined by a rate assay at 25°C.

Composition of the reactant solution:

83 mM Tris-HCl having a pH of 8.5
50 mM GEDTA
1.67 mM DL-isocitrate
1.0 mM $NADP^+$
0.25 u/ml iCDH (NADP)

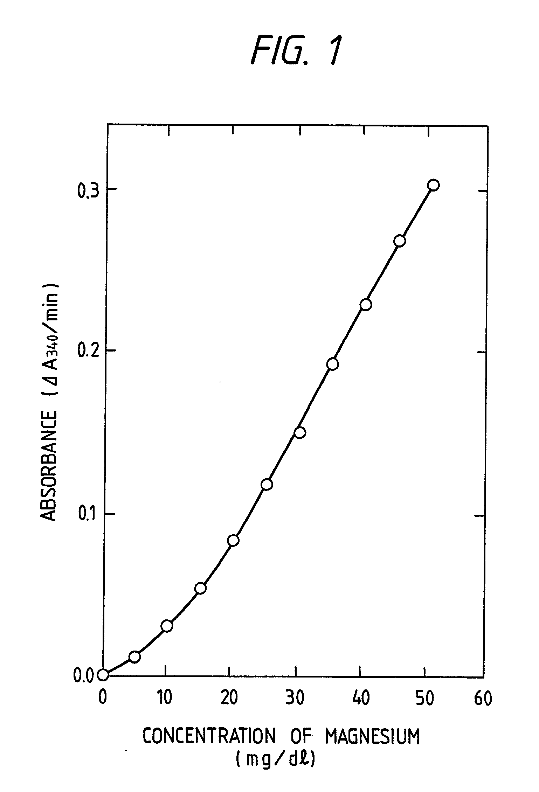

The results are shown in FIG. 1.

## Example 2

A standard solution containing 250 mg/dl of $Mg^{2+}$ was prepared by dissolving 42.3 mg of $MgCl_2 \cdot 6H_2O$ in purified water. The standard solution was diluted in various ways to make solutions containing 0, 1, 3, 5, 7 and 10 mg/dl, respectively, of $Mg^{2+}$.

Then, 50 μl of each solution was added to 3 ml of a reactant solution of the composition shown below, and a variation of absorbance at 340 nm was determined by a rate assay at 25° C.

Composition of the reactant solution:

83 mM Tris-HCl having a pH of 8.5
15 mM ATP
1.67 mM DL-isocitrate
1.0 mM $NADP^+$
0.8 u/ml iCDH (NADP)

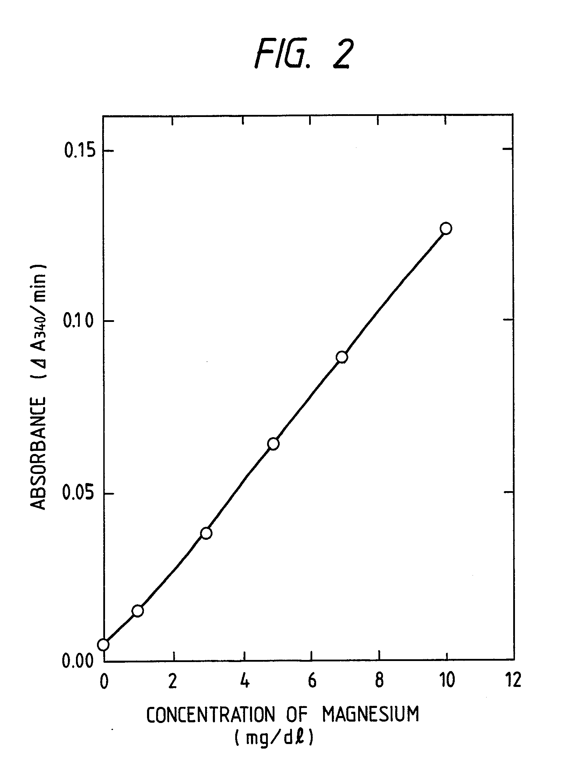

The results are shown in FIG. 2.

## Claims

1. A method of assaying magnesium which relies upon the reaction of isocitrate dehydrogenase to determine the quantity of magnesium.

2. A method as set forth in claim 1, wherein an agent for chelating magnesium ions and/or adenosine triphosphate (ATP) is added as an inhibitor to the reaction of isocitrate dehydrogenase.

3. A method as set forth in claim 1, wherein glycol ether diaminetetraacetic acid (GEDTA) is added as an agent for chelating manganese ions.

FIG. 1

FIG. 2

## European Patent Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-4657854 (P.F.WEGFAHRT) <br> * column 7, lines 1 - 7; claims 1, 9 * | 1 | G01N33/84 <br> C12Q1/32 |
| A | ANALYTICAL BIOCHEMISTRY <br> vol. 95, no. 1, 1979, NEW YORK US <br> pages 62 - 72; B.A.Bulos et al.: <br> "Determination of the concentration of free calcium ion in the presence of magnesium or mangenese and chelating effectors of the NAD-linked isocitrate dehydrogenase " <br> * the whole document * | 1-3 | |
| A | EP-A-286039 (THE FLINDERS UNIVERSITY OF SOUTH AUSTRALIA) <br> * page 3, lines 56 - 58 * <br> * page 4, lines 23 - 24 * <br> * page 4, lines 39 - 51; claims 1-13, 23 * | 1-3 | |
| A | EP-A-199363 (ORIENTAL YEAST CO. LTD.) <br> * page 6, line 7 - page 7, line 15; claim 1 * | 1, 2 | |
| A | CLINICAL CHEMISTRY. <br> vol. 32, no. 4, April 1986, WINSTON US <br> pages 629 - 632; M.C.Wimmer et al.: <br> "A kinetic colorimetric procedure for quantifying magnesium in serum" <br> * the whole document * | 1 | TECHNICAL FIELDS SEARCHED (Int. Cl.5 ) <br><br> G01N <br> C12Q |
| A | DE-A-1673197 (VEB ARZNEIMITTELWERK DRESDEN) <br> * the whole document * | 1 | |

The present search report has been drawn up for all claims

| Place of search | Date of completion of the search | Examiner |
|---|---|---|
| BERLIN | 08 FEBRUARY 1990 | DE KOK A.J. |